# EUROPÄISCHE PATENTSCHRIFT

(11) **EP 1 677 618 B1**
(45) Veröffentlichungstag und Bekanntmachung des Hinweises auf die Patenterteilung: **18.05.2011**
(21) Anmeldenummer: 04765881.0
(22) Anmeldetag: 08.10.2004
(51) Int. Cl.: A23G 3/00

(54) **GELATINEFREIE, ISOMALTULOSE-HALTIGE WEICHKARAMELLE**
GELATINE-FREE SOFT CARAMEL CONTAINING ISOMALTULOSE
CARAMELS MOUS RENFERMANT DE L'ISOMALTULOSE ET EXEMPTS DE GELATINE

(30) Priorität: 23.10.2003 DE 10349465
(43) Veröffentlichungstag der Anmeldung: 12.07.2006
(73) Patentinhaber: Südzucker Aktiengesellschaft Mannheim/Ochsenfurt, 68165 Mannheim (DE)
(72) Erfinder: BERNARD, Jörg, 67283 Albsheim (DE); KOWALCZYK, Jörg, 67304 Eisenberg/Steinborn (DE)
(74) Vertreter: Schwahn, Hartmut
(86) Internationale Anmeldenummer: PCT/EP2004/011233
(87) Internationale Veröffentlichungsnummer: WO 2005/044016

(56) Entgegenhaltungen:
- EP-A- 0 390 438
- EP-A- 0 979 611
- EP-A- 1 013 175
- EP-A- 1 023 841
- EP-A- 1 133 924
- WO-A-00/44241
- GB-A- 2 152 351
- US-A- 4 415 596
- US-A- 4 587 119
- US-A1- 2002 076 478
- US-B1- 6 555 146

## Beschreibung

Die vorliegende Erfindung betrifft gelatinefreie, Isomaltulose-haltige Weichkaramellen und Verfahren für deren Herstellung.

Gelatine ist eines der bekanntesten tierischen Erzeugnisse, das großtechnisch aus dem Kollagen vor allem aus Knochen und Häuten von Schlachttieren, insbesondere Rindern und Schweinen, gewonnen wird. Gelatine bildet in warmem Wasser eine visköse Lösung, die bei einer Gelatine-Konzentration von mindestens 1 Gew.-% unterhalb von etwa 35°C gallertartig erstarrt. Gelatine wird daher in vielen Lebensmitteln als Gelier-, Aufschlag- und Bindemittel, texturgebendes Mittel und/oder Emulgator eingesetzt. Gelatine zeichnet sich ebenfalls durch leichte Verdaulichkeit aus. Gelatine prägt Aussehen von beispielsweise Sülzen und Sülzwürsten, Geleespeisen und Gelee-Süßwaren. Bei Produkten wie beispielsweise Speiseeis und Joghurt-Erzeugnissen wirkt Gelatine konsistenzverbessernd.

In Süßwaren wie Weichkaramellen wird Gelatine ebenfalls als texturgebendes Mittel eingesetzt. Von Bedeutung ist hier insbesondere die Fähigkeit von Gelatine zur Bindung der Fettbestandteile. Darüber hinaus beeinflusst Gelatine die Kaufähigkeit der Weichkaramellenmasse, indem die Rekristallisation von Weichkaramellen-Bestandteilen, insbesondere Zucker-Arten vermindert beziehungsweise verhindert wird. Gelatine verhindert auch die Agglomeration, d.h. das Zusammenwachsen kleiner, sehr feiner Kristalle. Dabei werden die Gelatine-Moleküle von der Oberfläche der Kristalle absorbiert und bauen eine Art Isolierschicht um die Kristalle auf, wobei jedoch die Beschaffenheit der Kristalle selbst nicht verändert wird.

Des weiteren beeinflusst Gelatine auch das Schaumvermögen der Weichkaramellenmasse. Da Gelatine ein Hydrokolloid ist, wirkt sie durch Erhöhung der innerlamellaren Wasserbindung stabilisierend.

Gelatine-haltige Lebensmittel werden jedoch aus unterschiedlichen Gründen zumindest von bestimmten Konsumenten-Kreisen zunehmend abgelehnt. Beispielsweise verzehren viele Vegetarier, wenn überhaupt, nur tierische Produkte wie Milch, Milchprodukte und Eier, jedoch keine anderen vom Tier stammenden Produkte, also auch keine Gelatine-haltigen Lebensmittel. Auch Anhänger der koscheren Ernährungsweise, die beispielsweise in den Vereinigten Staaten relativ weit verbreitet ist und häufig auch von Konsumenten nichtjüdischen Glaubens praktiziert wird, lehnen den Verzehr Gelatine-haltiger Lebensmittel ab. Auch das Aufkommen, der Bovine Spongiform Encephalopathy (BSE)-Erkrankung bei Rindern hat die Nachfrage nach gelatinefreien Produkten stark erhöht. Nicht zuletzt sprechen auch ökonomische Gründen dafür, Gelatine durch alternative nicht-tierische Produkte, beispielsweise auf pflanzlicher Basis, zu ersetzen.

Bei Backwaren, Pudding-Produkten oder Joghurt wird Gelatine daher beispielsweise durch Agar-Agar ersetzt. Der üblicherweise eingesetzte Agar weist jedoch den Nachteil auf, dass er mehrer Minuten lang gekocht werden muss, damit die Produkte genügend Wasser einlagern und effizient weiterverarbeitet werden können. In Milchprodukten wie Brotaufstrichen, Desserts, Aufschlagprodukten und fermentierten Produkten werden beispielsweise Kombinationen pflanzlicher oder mikrobieller Hydrokolloiden eingesetzt, um eine gelatineartige Wirkung zu erreichen, insbesondere um eine bestimmte Textur zu erzielen, Synärese, also die Phasentrennung bei Gelen zu vermeiden und Schaumstabilität zu erreichen. Diese Kombinationen bestehen oft aus einem Gemisch aus gelierenden und nicht-gelierenden Substanzen. In Milchprodukten wie Joghurt werden häufig auch Stärken als Alternative zu Gelatine eingesetzt. Stärken bilden bei Erhitzung ebenfalls Gele und können Wasser speichern. Die Verwendung von Stärke oder Stärke-Derivaten als alleiniger Gelatine-Ersatz in Milchprodukten ist jedoch teilweise mit erheblichen Problemen behaftet, da für einige Produkte eine außerordentlich hohe Dosierung gewählt werden muss, um eine Gelierung zu bewirken. Für einige Milchprodukte sind daher Kombinationen von Stärken und Hydrokolloiden besser geeignet. In vielen Milchprodukten werden auch faserhaltige Produkte wie Oligofructose-Produkte und Weizenfaserprodukte, meist in Kombination mit Stärken, zur Verbesserung von Mundgefühl und Textur eingesetzt.

EP 1 023 841 A1 offenbart eine Gelatine-freie Weichkaramelle, die aus einer Weichkaramellengrundmasse besteht, die mindestens ein Polysaccharid-Hydrokolloid als Textur-gebende Struktur, eine kristalline Süßungsmittelphase (Isomalt) und eine nicht-kristalline Süßungsmittelphase enthält.

US 4587 119 offenbart eine Gelatine-freie Weichkaramelle (Toffee), die eine Weichkaramellengrundmasse, eine kristalline Süßungsmittelphase (Isomaltulose) und eine nicht-kristalline Süßungsmittelphase enthält.

Der Ersatz von Gelatine in Nahrungsmitteln ist trotz der vorstehend genannten Entwicklungen nach wie vor eine außerordentlich schwierige Aufgabe. Es hat sich herausgestellt, dass bei den meisten Anwendungen ein einzelner Zusatzstoff allein nicht die Eigenschaften aufweist, die für den vollständigen Ersatz von Gelatine erforderlich sind.

Das der vorliegenden Erfindung zugrunde liegende technische Problem besteht darin, gelatinefreie Weichkaramellen, wobei Gelatine durch eine nicht-tierische Substanz ersetzt wird, die Eigenschaften wie geringe Elastizität, hohe Wasser-Dispergierbarkeit, gute körpergebende und texturgebende Eigenschaften, Mundgefühl und keinen Eigengeschmack aufweist und daher Gelatine vollständig ersetzen kann, sowie Verfahren für deren Herstellung bereitzustellen.

Die vorliegende Erfindung löst das hier zugrundeliegende technische Problem durch die Bereitstellung einer gelatinefreien Weichkaramelle, umfassend eine Weichkaramellengrundmasse, die mindestens ein Polysaccharid-Hydrokolloid, ausgewählt aus der Gruppe bestehend aus: Gummi arabicum, Gellan-Gummi, Guar-Gummi, Cellulose-Gummi, Johannisbrotsamen-Gummi, Tamarindensamen-Gummi, Tara-Gummi, Tragant-Gummi, Xanthan-Gummi, Agar, Alginat, Carragen, Konjak, Pektin, Pullulan, Stärke, modifizierter Stärke und einem Gemisch davon, als texturgebendes Mittel, eine durch Isomaltulose gebildete kristalline Süßungsmittel-Phase und eine nicht-kristalline Süßungsmittel-Phase enthält.

Erfindungsgemäß wurde überraschenderweise festgestellt, dass Polysaccharid-Hydrokolloide solche Eigenschaften aufweisen, die den vollständigen Ersatz von Gelatine als texturgebendes Mittel in Weichkaramellen erlauben, so dass die besondere Textur und Konsistenz der Weichkaramellen erhalten bleibt.

Weichkaramellen besitzen eine weiche und kaufähige Konsistenz, die auf einen Restwassergehalt von 6 % bis 10% und auf für Weichkaramellen charakteristische Rezepturbestandteile wie Fett und bislang Gelatine zurückzuführen ist. Grundsätzlich bestehen Weichkaramellen aus einer weniger gut löslichen, kristallinen Phase, einer gut löslichen, nicht-kristallinen Phase und einer in der Weichkaramellenmasse eingeschlossene gasförmige Phase, die zu einer geschmeidigen und leichten Beschaffenheit führt. Die nicht-kristalline Phase dient in der Weichkaramellenmasse zur Hemmung der Kristallisation von Bestandteilen und zur Stabilisierung der Feuchtigkeit, wobei die nicht-kristalline Phase auch entscheidenden Anteil an der Körperbildung und der Festigkeit und Viskosität der Weichkaramellenmasse hat und die Kaufähigkeit der Weichkaramelle beeinflusst. Weichkaramellen umfassen auch eine flüssige Phase, deren Viskosität von ausschlaggebender Bedeutung für die Konsistenz der Weichkaramelle ist. In Verbindung mit Weichkaramellen-Bestandteilen wie Fett und Gelatine bewirken die Phasen die besondere Konsistenz der Weichkaramellen, insbesondere eine kaubare, kurze Textur, und regt den Konsumenten zum Kauen, nicht jedoch zum Lutschen, der Weichkaramellen an. Die Verwendung von Gelatine spielt bei der Herstellung herkömmlicher Weichkaramellen eine wichtige Rolle, da Gelatine als texturgebendes Mittel die Viskosität der Weichkaramellenmasse beeinflusst und dadurch bedingt die Rekristallisation von Weichkaramellen-Bestandteilen verhindert und sich ebenfalls positiv auf die Stabilisierung des Lufteinschlages auswirkt.

Erfindungsgemäß wurde nun festgestellt, dass Polysaccharid-Hydrokolloide in Weichkaramellen ebenso wie Gelatine Fett binden, Wasser speichern und den Lufteinschlag stabilisieren können, die Kaufähigkeit der erfindungsgemäßen Weichkaramelle durch die Verminderung beziehungsweise Verhinderung der Rekristallisation beeinflussen und die Agglomeration also das Zusammenwachsen von kleinen, sehr feine kristallförmigen Bestandteilen der erfindungsgemäßen Weichkaramelle verhindern Polysaccharid-Hydrokolloide beeinflussen auch in vorteilhafter Weise das Schaumvermögen der Weichkaramellenmasse und wirken dadurch stabilisierend. Darüber hinaus hat sich überraschenderweise herausgestellt, dass durch den erfindungsgemäßen Ersatz von Gelatine durch Polysaccharid-Hydrokolloide in einer Weichkaramelle auch die Temperaturstabilität der erfindungsgemäß als kristalline Phase eingesetzten Isomaltulose wesentlich verbessert.

Die-erfindungsgemäße gelatinefreie Weichkaramelle zeichnet- sich insbesondere auch dadurch aus, dass die herkömmlicherweise in Weichkaramellen verwendete Saccharose als kristalline Süßungsmittelphase sowohl in technologischer als auch in geschmacklicher Hinsicht vollständig durch Isomattulose ersetzt ist. Isomaltulose vermittelt der erfindungsgemäßen gelatinefreien Weichkaramelle somit einen süßen Geschmack, fördert die Geschmacksentfaltung der in der Weichkaramelle enthaltenen Aromen und trägt auch zur Körper Bildung der erfindungsgemäßen Weichkaramelle bei. Die erfindungsgemäß als kristalline Phase eingesetzte Isomaltulose zeichnet sich durch eine geringe Löslichkeit und eine damit im Zusammenhang stehende Kristallisationsneigung aus. Die starke Isomaltulose-Kristallisation führt in vorteilhafter Weise zur Texturverkürzung der Weichkaramellenmasse. Isomaltulose bewirkt daher neben anderen Bestandteilen der erfindungsgemäßen Weichkaramelle deren Plastizität und Textur.

Im Zusammenhang mit der vorliegenden Erfindung wird unter einer "Weichkaramelle" eine Süßware verstanden, die aus einem Sirups Fett und einer Süßungsmittel-Lösung unter Einkochen hergestellt wird. Herkömmliche Weichkaramellen weisen etwa 30-60% Saccharose, 20-50% Stärkesirup, 1-10% Invertzucker, 0-6% Lactose, 2-15% . Fett, 0-5% Milcheiweiß, 0-0,5% Gelatine und 4-8% Wasser auf. Weichkaramellen enthalten darüber hinaus Säuren und Aromastoffe. Die gegenüber Hartkaramellen wesentlich elastischere Konsistenz von Weichkaramellen wird durch den höheren Fett- und Wasseranteil sowie durch das Einschlagen von Luft erzielt. Als Fettkomponente für die Weichkaramellherstellung werden insbesondere Emulgator-haltige Triglyceride auf der Basis von Palmkern- oder Sojafett eingesetzt.

Im Zusammenhang mit der vorliegenden Erfindung werden unter "Hydrokolloiden" Dickungsmittel, Quellungsmittel oder Gelbildner verstanden, bei denen es sich um organische hochmolekulare Stoffe handelt, die Flüssigkeiten, in der Regel Wasser, aufnehmen und quellen können. Hydrokolloide gehen dabei in zähflüssige echte oder kolloidale Lösungen über und bilden dann Gele oder Schleime. Dickungsmittel beeinflussen weitgehend die Konsistenz eines Lebensmittels, beispielsweise durch Erhöhung der Viskosität eines Systems, Bildung einer Gelstruktur oder durch Herabsetzung der Oberflächenspannung. Dickungsmittel besitzen daher auch eine Emulgatorwirkung. Dickungsmittel können auf diese Weise Fest/Flüssig-Systeme wie Fruchtnektare, Flüssig/Flüssig-Systeme wie Dressings oder Gas/Flüssig-Systeme wie aufgeschlagene Milchprodukte stabilisieren. Dickungsmittel beeinflussen darüber hinaus auch die positiven und negativen Empfindungen, welche die Textur des Nahrungsmittel im Mund verursacht, und damit auch den Genusswert eines Nahrungsmittels. Weitere Wirkungen von Dickungsmitteln in Lebensmitteln sind die Verminderung von Wasserverlusten durch dessen Bindung und damit Verlängerung der Frischeperiode, die Verhinderung der Kristallisation von Lebensmittel-Inhaltsstoffen, zum Beispiel von Zucker-Arten, und die Verbesserung von mechanischen Eigenschaften von Lebensmitteln wie Festigkeit, Elastizität und Gashalteverrnögen.

Unter "Hydrokolloiden auf Polysaccharid-Basis" oder "Polysaccharid-Hydrokolloiden" werden im Zusammenhang mit der vorliegenden Erfindung Hydrokolloide verstanden, die aus Polysacchariden, insbesondere Polysacchariden pflanzlichen oder mikrobiellen Ursprungs bestehen. Polysaccharid-Hydrokolloide sind daher Stoffe, die in Wasser löslich oder nur dispergierbar sind und unter Wasseraufnahme quellen können, so dass visköse Lösung, Pseudogele oder Gele entstehen. Sie wirken beispielsweise durch die Versteifung der wässrigen Phase oder durch direkte Wechselwirkungen mit oberflächenaktiven Substanzen.

"Polysaccharide" sind makromolekulare Kohlenhydraten, deren Moleküle aus einer großen Zahl, insbesondere mindestens mehr als 10, normalerweise jedoch erheblich mehr, glycosidisch miteinander verknüpfter Monosaccharid-Moleküle bestehen. Polysaccharide können aus nur einer Art von Bausteinen bestehen, die gegebenenfalls in wechselnder glycosidischer Verknüpfung miteinander verbunden sind. Polysaccharide, insbesondere die in Pflanzengummen vorkommenden Heteroglykane, können auch aus verschiedenen Monomereinheiten bestehen.

Gemäß der Erfindung handelt es sich bei dem in Weichkaramellen als texturgebendes Mittel eingesetzten Polysaccharid-Hydrokolloid um Gummi arabicum, Gellan-Gummi, Guar-Gummi, Cellulose-Gummi, Johannisbrotsamen-Gummi, Tamarindensamen-Gummi, Tara-Gummi, Tragant-Gummi, Xanthan-Gummi, Agar-Agar, Alginate, Carrageen, Konjak, Pektin, Pullulan, Stärken, modifizierte Stärken oder Gemische davon.

"Gummi arabicum" ist das getrocknete Exsudat verschiedener Akazienarten. Bei Gummi arabicum handelt es sich um ein schwach saures Produkt, welches in natürlicher Form als neutrales oder schwach saures K-, Ca- oder Mg-Salz vorkommt. Die Hauptbestandteile von Gummi arabicum sind L-Arabinose, L-Rhamnose, D-Galactose und D-Glucuronsäure. Die molaren Verhältnisse dieser Bestandteile sind stark abhängig von der Akazienart, aus der Gummi arabicum gewonnen wird. Gummi arabicum ist ein verzweigtes Polysaccharid, dessen Hauptteile aus β-(1,3)-verzweigten D-Galactopyranose-Einheiten besteht. Gummi arabicum ist sehr gut wasserlöslich, wobei 1-15%-ige Lösungen nur geringe Viskosität besitzen, während höhere Konzentrationen zu einer zähen, gelartigen Masse führen.

"Gellan-Gummi" ist ein extrazelluläres Polysaccharid des Organismus Sphingomonas elodea. Das hochmolekulare Polysaccharid besteht prinzipiell aus einer sich wiederholenden Tetrasaccharid-Einheit die ein Rhamnose-Molekül, zwei Glucuronsäure-Moleküle und zwei Glukose-Moleküle umfasst, und ist mit Acyl-Gruppen, insbesondere Glyceryl- und Acetyl-Gruppen, substituiert. Gellan-Gummi kann unterschiedliche Texturen bilden, die von weichen elastischen Gelen bis zu starren spröden Gelen reichen. Durch das Mischen von Gellan-Gummis mit einem hohen Anteil von Acyl-Gruppen und Gellan-Gümmis mit einem geringen Anteil von Acyl-Gruppen lassen sich Gel-Tekturen unterschiedlichster Art herstellen.

"Guar-Gummi" ist ein kolloides Pulver, das durch Mahlen des Endosperms der Samen des Baumes Cyamopsis tetragonolobus gewonnen wird. Der lösliche Teil des Guar-Gummis ist ein nichtionogenes Polysaccharid aus β-1,4-glycosidisch verknüpften D-Mannopyranose-Einheiten mit α-1,6-verknüpfter D-Galactopyranose in der Seitenkette und zwar je eine D-Galactose-Einheit pro 2 Mannose-Einheiten. Guar-Gummi quillt als Hydrokolloid in Wasser auf, ohne jedoch eine klare Lösung zu bilden. Bei Zusatz einer kleinen Menge von Borax zur Guar-Gummi Lösung entstehen gummiartig Gele. Mit anderen Polysacchariden wie Agar, Carrageen, Stärke oder Xanthan zeigt Guar-Gummi synergistische Effekte.

"Cellulose-Gummis" werden durch chemische Modifikation von Cellulose, einem linearen, Glucose-basierten Polymer mit β-1,4-Bindungen, erhalten. Cellulose-Gummis umfassen mikrokristalline Cellulose (MCC), Carboxymethylcellulose (CMC), Methylcellulose (MC) und Hydroxypropylmethylcellulose (HPMC). Durch Hydrolyse von Cellulose werden MCC-Kristalle in pulverförmiger oder kolloidaler Form erhalten. Obwohl diese Kristalle nicht löslich sind, kann die kolloidale Form unter Bildung thixotroper Gele Wasser aufnehmen. Die so gebildeten Gele können als Stabilisierungsmittel oder Fettersatzmittel eingesetzt werden. CMC ist das Natriumsalz des Carboxymethylethers von Cellulose mit einem Substitutionsgrad von 0,4 bis 0,8. Der Substitutionsgrad beeinflusst die Eigenschaften des Gummis, einschließlich dessen Löslichkeit. CMC kann Protein-Dispersionen stabilisieren. Durch Umsetzung von Alkali-Cellulose mit Methylchlorid wird MC gebildet, während die Umsetzung von Alkali-.. Cellulose mit Propylenoxid und Methylchlorid zur Erzeugung von HPMC führt. Die in kaltem Wasser lösliche Methylcellulosen zeigen eine reversible thermische Gelierung, dass heißt unter Hitzeeinfluss gelieren sie, während es bei verminderten Temperaturen zu einer Resolubilisierung kommt. Ebenso wie CMC beeinflusst DS die Eigenschaften des Gummis, so dass bei Temperaturen von 50°C gebildete feste Gele bei Temperaturen von mehr als 90°C in schwache Gele übergehen.

"Johannisbrotsamen-Gummi" (Johannisbrotkernmehl) ist ein Galactomannan aus dem Endosperm der Samen des Johannisbrotbaumes. Der Gummi weist ein Molekulargewicht von 300 000 bis 360 000 auf und besteht aus einer Kette aus β-(1,4)-verknüpften D-Mannopyranosid-Einheiten, an welchen α-(1,6)-verknüpfte α-Galactopyranosid-Einheiten haften, wobei der Gehalt Mannose/Galactose zwischen 5:1 bis 4:1 liegt. Vermutlich existieren im Molekül Blöcke unsubstituierte Mannose-Einheiten, zwischen denen Bereiche liegen, in denen jeder zweite Mannose-Rest einer Galactose-Einheit trägt.

"Tamarindensamen-Gummi" (Tamarindenkernmehl) ist ein aus den Samen der Tamarinde gewonnenes Hydrokolloid, dass aus β-(1,4)-verknüpften D-Glucose-Einheiten in der Hauptkette und D-Xylose, D-Galactose und L-Arabinose in den Verzweigungen besteht. Das Molekulargewicht beträgt etwa 50 000. Tamarindenkeermehl ergibt in kaltem Wasser hochvisköse Lösungen, die auch ohne Säure mit 65-72% Saccharose gelieren. Tamarindenkernmehl bildet über einen breiten pH-Bereich stabile Gele. Diese zeigen nur eine geringe Synärese. Die Gele sind im Vergleich zu Pektin auch bei geringeren Zuckerkonzentrationen stabil.

Tara-Gummi ist ein im Endosperm des Samens des Tara-baumes vorkommendes Galactomannan mit den Einzelbausteinen Galactose und Mannose im Verhältnis 1:3. Das Molekül besteht aus β-(1,4)-verknüpften D-Mannopyranose-Einheiten, an die sich seitlich D-galactopyranosid-Einheiten und α-(1,6) Bindungen anheften. Ober die Verteilung der Galactose-Moleküle in der Kette gibt es noch keine Klarheit. Die physikalischen und chemischen Eigenschaften entsprechen weitestgehend denen von Guar-Gummi und Johannisbrotkernmehl. Tara-Gummi ist in kaltem Wasser nicht vollständig löslich, wobei die Lösung eine signifikant höherer Viskosität als gleich konzentrierte Lösungen von Guar-Gummi oder Johannisbrotkernmehl besitzen. Ähnlich wie Johannisbrotkernmehl bildet Tara-Gummi mit Xanthan Gele, nur sind letztere schwächer und die Schmelzpunkte der Gele tiefer liegen. Auch bei Agar und Carrageen zeigt Tara-Gummi synergistische Gelverstärkungen.

"Tragant-Gummi" ist ein Exsudat aus Stämmen und Zweigen der zu den Astragalus-Arten gehörenden Sträucher. Die Einzelbausteine von Tragant-Gummi sind L-Rhamnose, L-Fucose, D-Xylose, L-Arabinose, D-Galactose, D-Glucose und D-Galacturonsäure im Verhältnis 2,0 : 2,8 : 8,3 : 24,5 : 7,0 : 7,6 : 23,2. Tragant-Gummi besteht zu 60-70 % aus einem in Wasser quellbaren, aber nicht löslichen Anteil (Bassorin) und zu 30-40 % aus einer wasserlöslichen Fraktion, dem sogenannten Tragacanthin. Beim wasserlöslichen Anteil handelt es sich um ein hoch verzweigtes Arabinogalactan, bestehend aus 75 % L-Arabinose, 10 % D-Galactose und 10 % D-Galacturonsäure. Bei Bassorin handelt es sich um ein stark verbzweigtes Molekül mit einer Kette aus α-(1,4)-verknüpften D-Galacturonsäuren, welche in C3-Positionen unterschiedlich lange Seitenketten tragen. Tragant quillt in Wasser unter Aufnahme einer Wassermenge, die dem 45-50-fachen des eigenen Gewichtes entspricht, wobei es zur Bildung von zähen hochviskösen Schleimen kommt, die in einem pH-Bereich von 2-8 in der Konsistenz beständig sind.

"Xanthan-Gummi" ist ein extrazelluläres Heteropolysaccharid von Xanthomonas campestris mit den Einzelbausteinen D-Glucose, D-Mannose und D-Glucuronsäure im Verhältnis 2,8:2,0:2,0. Daneben enthält es noch etwa 5 % Acetyl- und 3 % Pyruvyl-Gruppen. Es handelt sich um eine -β-(1,4)-Glucankette; bei welcher die 3-Position des Glucose-Moleküls mit einer Seitenkette verbunden ist, die aus zwei Mannose-Einheiten und einer Glucuronsäure-Einheit besteht. Xanthan-Gummi ist in kaltem und heißem Wasser leicht löslich und weist eine hohe Pseudoplastizität auf. Mit dreiwertigen Kationen ist Xanthan-Gummi aus der Lösung fällbar. Xanthan-Gummi wird durch menschliche Verdauungsenzyme nicht abgebaut und im Dickdarm durch dort angesiedelte Mikroorganismen teilweise zerlegt.

"Agar" (Agar-Agar) ist ein Polysaccharid aus den Zellwänden zahlreicher Rotalgen der Spezies Gelidium und Gracilaria. Agar ist ein Gemisch aus der gelierenden Agarose, einem linearen Polysaccharid mit einem Anteil bis zu 70 %, und dem nicht-gelierenden Agaropektin (β-1,3-verknüpfte D-Galactose-Einheiten) mit einem Anteil von bis zu 30 %. Das Molekulargewicht von Agar beträgt etwa 110000 - 160000. Agar ist in kaltem Wasser unlöslich, jedoch in heißem Wasser löslich. Noch mit einer 1 %-igen Lösung wird ein festes Gel gebildet, das bei 80-100°C schmilzt und sich bei 45°C wieder verfestigt:

Bei "Alginaten" handelte es sich um Salze der Alginsäure. Alginate sind saure, Carboxy-Gruppen enthaltende Polysaccharide mit einem Molekulargewicht von ca. 200.000, bestehend aus D-Mannuronsäure und L-Guluronsäure in unterschiedlichen Verhältnissen, welche mit 1,4-Glycosidbindungen untereinander verknüpft sind. Die Na-, K-, NH₄- und Mg-Alginate sind wasserlöslich. Ca-Alginate bilden bei bestimmten Mengenverhältnissen thermoirreversible Gele. Durch Ansäuern mit Mineralsäuren von wässrigen Alginat-Lösungen scheidet sich die wasserunlösliche Alginsäure aus. Alginate können insbesondere das Auskristallisieren von Zucker beziehungsweise Zucker-arten verhindern.

"Carrageen" ist eine Gruppe von Polysacchariden, die in einer Reihe von Rotalgen enthalten sind. Hinsichtlich der chemischen Struktur ist Carrageen ähnlich wie Agar aufgebaut, wobei man verschiedene Fraktionen der Galactosesulfate unterscheidet. Kommerziell bedeutungsvoll sind 1-Carrageenan, κ-Carrageenan und τ-Carrageenan. λ-Carrageenan ist ein Kettenmolekül, das aus dimeren Bausteinen, nämlich β-1,3-D-Galactose-4-sulfat und α-1,4-3,6-D-Anhydrogalactose aufgebaut ist. Diese Dimere sind bei 1,3-glycosidisch verknüpft. Die primäre Alkoholgruppe der α-D-Galactose ist mit Schwefelsäure verestert und die Hydroxy-Gruppen an der C2-Position beider Galactose-Reste sind ebenfalls zu etwa 70 % mit Schwefelsäure verestert.

κ- und τ-Carrageenan sind aus dem Dimer Carrabiose aufgebaut, in welchem β-D-Galactose 1,4-glycosidisch an α-D-3,6-Anhydrogalactose gebunden ist. Diese Dimere sind durch 1,3-glycosidische Bindungen zu einem Kettenmolekül verknüpft. Der Tunterschied zwischen den beiden Carrageenan-Typen liegt in der Sulfatierung. Während sich beim κ-Carrageenan die Sulftatester-Gruppe am C4 der Galactose befindet, ist beim τ-Carrageenan zusätzlich die Hydroxy-Gruppe am C2 der Anhydrogalactose mit Schwefelsäure verestert. Das durchschnittliche Molekulargewicht von Carrageenan liegt zwischen 100.000 und 800.000.

"Konjac" ist ein Glucomannan, das aus den Wurzeln von A-morphophallus konjac gewonnen wird. Konjac ist ein aus Mannose und Glucose aufgebautes lineares Molekül mit nach dem Zufallsprinzip verteilten Acetyl-Gruppen. In pulverförmiger Form quillt es bei niedrigen Temperaturen langsam auf. Es bildet ein elastisches thermisch irreversibles Gel bei Behandlung mit Alkali und Hitze, wobei das Gel bei einem pH-Wert von 3 bis 9 stabil ist. "Pektine" sind in allen höheren Pflanzen weit verbreitet und werden insbesondere aus den Schalen von Zitrusfrüchten und Apfeltrester extrahiert. Haupteinzelbausteine von Pektinen sind D-Galacturonsäure. Daneben finden sich als Nebenbestandteile L-Rhamnose, D-Galactose, L-Arabinose und D-Xylose. Das Pektin-Molekül besteht aus einer Kette von (1,4)-verknüpften α-D-Galacturonsäure-Einheiten, die durch L-Rhamnose-Einheiten, welche 1-2 Position miteinander verknüpft sind, unterbrochen werden. Daran können sich noch als Seitenketten D-Galactose-, D-Xylose- und L-Arabinose-Einheiten anheften. Die Molmasse extrahierter Pektine beträgt durchschnittlich 100.000 und ist stark von den jeweilig angewendeten Extraktionsbedingungen abhängig. Hoch und niedrig veresterte Pektine sowie die Alkalisalze der Pektinsäure sind löslich in Wasser, während Pektinsäure in Wasser unlöslich ist. Durch Wasserstoffbrücken-Bildung kommt es in Teilbereichen der Pektin Kette zu Assoziationen, wobei sich dreidimensionale Netzwerke ausbilden.

"Pullulan" ist ein extrazelluläre Polysaccharid des hefeähnlichen Pilzes Aureobasidium pullulans. Pullulan ist ein Homopolysaccharid mit D-Glucose als einzigem Baustein, In Ketten sind Maltotriose-Einheiten durch α-1,6-Bindungen miteinander verbunden. Das Molekulargewicht von Pullulan beträgt 10.000-400.000.

In einer besonders bevorzugten Ausführungsform der Erfindung wird als Polysaccharid-Hydrokolloid ein Gemisch aus Gummi arabicum rund Gellan-Gummi eingesetzt. Vorzugsweise beträgt das Verhältnis zwischen Gummi arabicum und Gellan-Gummi 5:1 bis 15:1. Erfindungsgemäß ist vorgesehen, dass der Anteil des Polysaccharid-Hydrokolloids beziehungsweise Gemisches davon an der Gesamtmenge der Weichkaramellengrundmasse etwa 0,4 % bis etwa 0,8 % vorzugsweise etwa 0,6 %, bezogen auf das Trockengewicht der Weichkaramellengrundmasse, beträgt.

Erfindungsgemäß ist ferner vorgesehen, dass der Anteil der die kristalline Phase bildenden Isomaltulose an der Gesamtmenge der Weichkaramellengrundmasse etwa 35 % bis etwa 70 %, vorzugsweise etwa 42 %, bis etwa 65 %, bezogen auf das Trockengewicht der Weichkaramellengrundmasse, beträgt.

In einer weiteren bevorzugten Ausführungsform der Erfindung handelt es sich bei der gelatinefreien Weichkaramelle um eine zuckerfreie gelatinefreie Weichkaramelle, wobei die nicht-kristalline Süßungsmittel-Phase der Weichkaramellengrundmasse aus Maltitsirup, Polydextrose und/oder hydriertem Stärkehydrolysat gebildet wird. In einer weiteren bevorzugten Ausführungsform der Erfindung ist die erfindungsgemäße gelatinefreie Weichkaramelle eine zuckerhaltige gelatinefreie Weichkaramelle, wobei die nicht-kristalline Süßungsmittel-Phase der Weichkaramellengrundmasse aus Glucosesirup oder Stärkehydrolysat gebildet wird.

Erfindungsgemäß ist ebenfalls vorgesehen, dass die erfindungsgemäßen gelatinefreien Weichkaramellen neben den vorstehend genannten Zucker-Arten und/oder Zuckeraustauschstoffen zusätzlich einen oder mehrere Intensivsüßstoffe enthalten künnen. lntensivsüßstoffe sind Verbindungen, die sich bei geringem beziehungsweise vernachlässigbar geringem Nährwert durch einen intensiven Süßgeschmack auszeichnen. Erfindungsgemäß ist insbesondere vorgesehen, dass der Intensivsüßstoff Cyclamat, beispielsweise Natriumcyclamat, Saccharin, Aspartam, Glycyrrhizin, Neohesperidin-Dihydrochalcon, Thaumatin, Monellin, Acesulfam, Alitam oder Sucralose ist.

Erfindungsgemäß ist ferner vorgesehen, dass die Weichkaramellengrundmasse der gelatinefreien Weichkaramalle 2-15 % Fett enthält. Vorzugsweise handelt es sich bei dem in der erfindungsgemäßen gelatinefreien Weichkaramelle enthaltenen Fett um gehärtetes Palmkernfett.

In einer weiteren Ausführungsform der Erfindung ist vorgesehen, dass die Weichkaramellengrundmasse der gelatinefreien Weichkaramelle mindestens ein Emulgiermittel enthält. Unter einem "Emulgiermittel" oder "Emulgator" wird ein Hilfsstoff verstanden, der bei der Herstellung und zur Stabilisierung von Emulsionen eingesetzt werden. Emulgatoren sind oberflächenaktive Stoffe, die die Grenzflächenspannung zwischen den beiden Phasen Öl und Wasser herabsetzen und neben der Verringerung der Grenzflächenarbeit auch eine Stabilisierung mit der gebildeten Emulsion bewirken. Emulgatoren stabilisieren die gebildete Emulsion durch Grenzflächenfilme sowie durch Ausbildung sterischer oder elektrischer Barrieren, wodurch das Zusammenfließen der emulgierten Teilchen verhindert wird. Sowohl Elastizität als auch Viskosität der Grenzflächenfilme sind wichtige Faktoren der Emulsionsstabilisierung und werden stark vom Emulgator beeinflusst.

In einer weiteren Ausführungsform der Erfindung ist vorgesehen, dass die Weichkaramellengrundmasse der gelatinefreien Weichkaramelle 0 % bis 5 % mindestens eines Protein-Bestandteils enthält. Bei dem Protein-Bestandteil kann es sich erfindungsgemäß um ein Protein tierischen, pflanzlichen oder mikrobiellen Ursprungs handeln. Vorzugsweise handelt es sich bei dem Protein-Bestandteil insbesondere um Milcheiweiß.

In noch einer weiteren Ausführungsform der Erfindung ist vorgesehen, dass die Weichkaramellengrundmasse der erfindungsgemäßen gelatinefreien Weichkaramelle einen oder mehrere natürliche oder synthetische Lebensmittelfarbstoffe enthält. Im Zusammenhang mit der vorliegenden Erfindung wird unter einem "Lebensmittelfarbstoff" eine Substanz verstanden, die bei der Herstellung von Lebensmitteln zum Zwecke der Farbkorrektur oder zur Erzeugung eines ansprechenden Aussehens verwendet wird. Lebensmittelfarbstoffe leisten eine erheblichen Beitrag zur Akzeptanz von Lebensmitteln.Die erfindungsgemäß eingesetzten Lebensmittelfarbstoffe können sowohl natürlichen als auch synthetischen Ursprungs seien. Zu den natürlichen Lebensmittelfarbstoffen gehören Farbstoffe pflanzlicher Herkunft, beispielsweise Carotinoide, Flavonoide und Anthocyane, Farbstoffe tierischer Herkunft, beispielsweise Cochenille, sowie anorganische Pigmente wie Titanoxid, Eisenoxid-Pigmente und Eisenhydroxid-Pigmente. Zu den Lebensmittelfarbstoffen gehören auch Produkte der enzymatischen Bräunung wie Polyphenole und Produkte der nicht-enzymatischen Bräunung wie Melanoidine sowie Erhitzungsprodukte wie zum Beispiel Zuckercouleur und Karamelle. Zu den synthetischen Lebensmittelfarbstoffen gehören insbesondere Azo-, Triphenylmethan-, Indigoid-, Xanthen- und Chinolin-Verbindungen.

In bevorzugter Ausführungsform der Erfindung handelt es sich bei den Farbstoffen um Chlorophyll, ein Chlorophyllin, Karminrot, Alurarot, β-Karotin, Riboflavine, Anthocyane, Betanin, Erythrosin, Indigo Camine, Tartrazin oder Titanoxid.

Selbstverständlich kann die Weichkaramellengrundmasse der erfindungsgemäßen gelatinefreien Weichkaramelle zusätzliche Aroma- und Geschmacksstoffe enthalten. Solche Stoffe sind beispielsweise ätherische Öle, synthetische Aromen oder Gemische davon, beispielsweise Öle aus Pflanzen oder Früchten wie Citrusöl, Fruchtessenzen, Pfefferminzöl, Nelkenöl, Anis, kristalline Säure, Menthol, Eukalyptus etc.

Erfindungsgemäß ist vorgesehen, dass der Wassergehalt der Weichkaramellenmasse der erfindungsgemäßen gelatinefreien Weichkaramelle 5 bis 14 % Wasser, insbesondere 6 bis 12 % Wasser, vorzugsweise 6 bis 8 % beträgt.

In einer weiteren Ausführungsform ist vorgesehen, dass die Weichkaramellengrundmasse der erfindungsgemäßen gelatinefreien Weichkaramelle zusätzlich einen medizinischen Wirkstoff, beispielsweise Dextromethorphan, Hexylresorcin/Menthol, Phenylpropanolamin, Dyclonin, Mentholeukalyptus, Benzocain oder Cetylpyridinium enthält.

Die erfindungsgemäßen gelatinefreien Weichkaramellen können sowohl ungefüllt als auch gefüllt vorliegen, wobei die erfindungsgemäßen Weichkaramellen alle im Stand der Technik bekannten Füllungen aufweisen können. Selbstverständlich können die erfindungsgemäßen gelatinefreien Weichkaramellen ebenfalls dragiert oder undragiert vorliegen, wobei die im Stand der Technik üblicherweise zur Herstellung dragierter Weichkaramellen verwendeten Drageedecken eingesetzt werden können.

Die vorliegende Erfindung löst das ihr zugrunde liegende technische Problem auch durch ein Verfahren zur Herstellung einer gelatinefreien, Isomaltulose enthaltenden Weichkaramelle umfassend
a) Herstellung einer nicht-kristallinen Süßungsmittel-Phase durch Lösen mindestens eines löslichen Süßungsmittels in Wasser,
b) Zugabe mindestens eines Polysaccharid-Hydrokolloides, ausgewählt aus der Gruppe bestehend aus Gummi arabicum, Gellan-Gummi, Guar-Gummi, Cellulose-Gummi, Johannisbrotsamen-Gummi, Tamarindensamen-Gummi, Tara-Gummi, Tragant-Gummi, Xanthan-Gummi, Agar, Alginat, Carrageen, Konjak, Pektin, Pullulan, Stärke, modifizierter Stärke und einem Gemisch davon, mindestens eines Fettbestandteiles, mindestens eines Emulgiermittels sowie eines Teils der Gesamtmenge der die kristalline Süßungsmittel-Phase bildenden Isomaltulose zu der nicht-kristallinen Süßungsmittel-Phase,
c) Erhitzen des bei b) erhaltenen Gemisches auf eine Temperatur von mindestens 100°C unter Dampfzufuhr,
d) Zugabe der restlichen Isomaltulose zu dem erhitzten Gemisch unter Rühren,
e) Einführen von Luft in das bei d) erhaltene Gemisch und
f) Kühlen des Gemisches.

In einer bevorzugten Ausführungsform der Erfindung ist vorgesehen, dass etwa 70 % bis 90 % der Isomaltulose-Gesamtmenge der hergestellten nicht-kristallinen Süßungsmittelphase zugegeben und gemeinsam damit erhitzt werden. Vorzugsweise werden etwa 74 % bis 85 % der Isomaltulose-Gesamtmenge der nicht-kristallinen Süßungsmittelphase zugegeben und gemeinsam damit erhitzt.

In bevorzugter Ausführungsform wird das durch Mischen der nicht-kristallinen Süßungsmittel-Phase und des Fettbestandteils, des Polysaccharid-Hydrokolloides, des Emulgiermittels und eines Teils der Gesamtmenge von isomaltulose gebildete Gemisch auf eine Temperatur von 110°C erhitzt wird. In bevorzugter Ausführungsform wird nach Erhitzen des die nicht-kristalline Süßungsmittel-Phase enthaltenen Gemisches die Dampfzufuhr beendet und das Gemisch einem Vakuum unterworfen. Nach Beendigung der Dampftemperatur steigt die Temperatur des Gemisches dann auf 125°C bis 130°C. Anschließend wird dann der vorzugsweise zum Kochen des Gemisches verwendete Satzkocher geöffnet und die restliche Isomaltulose wird unter Rühren zu dem erhitzten Gemisch gegeben. Das Einführen von Luft in das so erhaltene Gemisch kann erfolgen, indem nach Zugabe der restlichen Isomaltulose die Luft durch Aufschlagen des erhitzten Gemisches in dieses eingeführt wird. In einer alternativen Ausführungsform wird das durch Zugabe der restlichen Isomaltulose erhaltene Gemisch zunächst abgekühlt und dann wird die Luft durch Ziehen des abgekühlten Gemisches in dieses eingeführt. Anschließend wird aus der aufgeschlagenen abgekühlten Masse beziehungsweise der gezogenen abgekühlten Masse ein Strang gezogen, von dem dann die entsprechenden Weichkaramellen-Stücke in gewünschter Größe geschnitten werden. Vorzugsweise weisen die abgeschnittenen Stücke ein Gewicht von 2 bis 7 g auf. Die so erhaltenen Weichkaramellen können dann unter Verwendung von für Weichkaramellen üblichen Verfahren, beispielsweise Wickeln oder Einschlagen, verpackt werden.

Die vorliegende Erfindung wird durch die folgenden Beispiele näher erläutert.

### Beispiel 1

### Herstellung von gelatinefreien lsomaltulose-haltigen Weichkaramellen

| **Rohstoffe I** | **g in Batch** | **in %** |
|---|---|---|
| Wasser | 267,00 | 7,93 |
| Polydextrose-Lösung mit 75 % TS | 1753,54 | 52,06 |
| Gehärtetes Palmkernfett | 192,00 | 5,70 |
| Gummi arabicum | 14,44 | 0,43 |
| Gellan-Gummi | 1,60 | 0,05 |
| Isomaltulose | 832,89 | 24,73 |
| Emulgator E 471 | 19,50 | 0,58 |
| Aspartam/Acesulfam K | 0,32 | 0,01 |
| | | |

| **Rohstoffe II** | | |
|---|---|---|
| lsomaltulose, feingemahlen, Typ PF | 287,24 | 8,53 |
| **Summe:** | **3368,53** | **100,00** |

Polydextrosepulver und Wasser werden mit einem Schneebesen vermischt. Danach werden alle anderen Zutaten der Rohstoffklasse I in einen Satzkocher gegeben und mit einem Rührwerk 3 Minuten herrührt. Anschließend wird die Masse erhitzt. Bei 110°C wird die Dampfzufuhr unterbrochen und anschließend wird für etwa 2 Minuten ein Vakuum angelegt. Nach Abstellen des Dampfes erwärmt sich die Masse weiter bis auf 125°C bis 130°C. Danach wird der Satzkocher geöffnet. Die Zutaten der Rohstoffklasse II werden hinzugegeben und für 3 Minuten mit einem Rührwerk untergerührt. Zum Abkühlen wird die Masse auf einen Kühltisch gegeben. Nach Abkühlen wird die Masse mit einer Ziehmaschine etwa 3 Minuten gezogen, um Luft einzuschlagen. Danach wird aus der gezogenen Masse ein Strang gezogen, von dem Stücke von etwa 2 bis 7 g abgeschnitten werden. Die so erhaltenen Weichkaramellen können mit den für Weichkaramellen üblichen Verfahren, zum Beispiel Wickeln oder Einschlagen, verpackt werden. Der Wassergehalt der durch dieses Verfahren erhaltenen Weichkaramellen liegt bei 6 bis 12 g/100 g Gesamtmenge.

### Beispiel 2

### Herstellung von gelatinefreien Weichkaramellen

| **Rohstoffe I** | **g in Batch** | **in %** |
|---|---|---|
| Wasser | 267,00 | 7,92 |
| Polydextrose-Lösung mit 75 % TS | 1034,57 | 30,70 |
| Gehärtetes Palmkernfett | 192,00 | 5,70 |
| Gummi arabicum | 16,04 | 0,48 |
| Gellan-Gummi | 1,60 | 0,05 |
| Isomaltulose | 1551,86 | 46,05 |
| Emulgator E 471 | 19,50 | 0,58 |
| Aspartam/Acesulfam K | 0,32 | 0,01 |
| | | |

| **Rohstoffe II** | | |
|---|---|---|
| Isomaltulose, feingemahlen, Typ PF | 287,24 | 8,52 |
| **Summe:** | **3370,13** | **100,00** |

Die gelatinefreien Weichkaramellen wurden analog zu Beispiel 1 hergestellt.

## Patentansprüche

1. Gelatinefreie Weichkaramelle, umfassend eine Weichkaramellengrundmasse, die
- mindestens ein Polysaccharid-Hydrokolloid, ausgewählt aus der Gruppe bestehend aus: Gummi arabicum, Gellan-Gummi, Guar-Gummi, Cellulose-Gummi, Johannisbrotsamen-Gummi, Tamarindensamen-Gummi, Tara-Gummi, Tragant-Gummi, Xanthan-Gummi. Agar, Alginat, Carrageen, Konjak, Pektin, Pullulan, Stärke, modifizierter Stärke und einem Gemisch davon, als texturgebendes Mittel,
- eine durch Isomaltulose gebildete kristalline Süßungsmittel-Phase und
- eine nicht-kristalline Süßungsmittel-Phase enthält.

2. Gelatinefreie Weichkaramelle nach Anspruch 1, wobei das Polysaccharid-Hydrokolloid ein Gemisch aus Gummi arabicum und Gellan-Gummi ist.

3. Gelatinefreie Weichkaramelle nach Anspruch 2, wobei Gummi arabicum und Gellan-Gummi in einem Verhältnis von 5:1 bis 15:1 im Gemisch vorliegen.

4. Zuckerfreie gelatinefreie Weichkaramelle nach einem der Ansprüche 1 bis 3, wobei die nicht-kristalline Süßungsmittel-Phase der Weichkaramellengrundmasse aus Maltitsirup, Polydextrose und/oder hydriertem Stärkehydrolysat gebildet wird.

5. Zuckerhaltige gelatinefreie Weichkaramelle nach einem der Ansprüche 1 bis 3, wobei die nicht-kristalline Süßungsmittel-Phase der Weichkaramellengrundmasse aus Glucosesirup und/oder Stärkehydrolysat gebildet wird.

6. Gelatinefreie Weichkaramelle nach einem der Ansprüche 1 bis 5, wobei die Weichkaramellengrundmasse zusätzlich einen oder mehrere Intensivsüßstoffe enthält.

7. Gelatinefreie Weichkaramelle nach Anspruch 6, wobei der Intensivsüßstoff Cyclamat, Saccharin, Aspartam, Glycyrrhizin, Neohesperidin-Dihydrochalcon, Thaumatin, Monellin, Acesulfam, Alitam oder Sucralose ist.

8. Gelatinefreie Weichkaramelle nach einem der Ansprüche 1 bis 7, wobei die Weichkaramellengrundmasse 2 bis 15% Fett enthält.

9. Gelatinefreie Weichkaramelle nach einem der Ansprüche 1 bis 8, wobei die Weichkaramellengrundmasse mindestens ein Emulgiermittel enthält.

10. Gelatinefreie Weichkaramelle nach einem der Ansprüche 1 bis 9, wobei die Weichkaramellengrundmasse 0 bis 5% mindestens eines Protein-Bestandteils, insbesondere Milcheiweiß, enthält.

11. Gelatinefreie Weichkaramelle nach einem der Ansprüche 1 bis 10, wobei die Weichkaramellengrundmasse einen oder mehrere natürliche oder synthetische Lebensmittelfarbstoffe enthält.

12. Gelatinefreie Weichkaramelle nach Anspruch 11, wobei der Lebensmittelfarbstoff Chlorophyll, ein Chlorophyllin, Karminrot, Alurarot, β -Karotin, ein Riboflavin, ein Anthocyan, Betanin, Erythrosin, Indigo Camine, Tartrazin oder Titandioxid ist.

13. Gelatinefreie Weichkaramelle nach einem der Ansprüche 1 bis 12, wobei die Weichkaramellengrundmasse Aroma- und Geschmacksstoffe enthält.

14. Gelatinefreie Weichkaramelle nach Anspruch 13, wobei die Aroma- und Geschmacksstoffe ätherische Öle, synthetische Aromen, Fruchtessenzen, Eukalyptus, Pfefferminzöl, Menthol und Säuren sind.

15. Gelatinefreie Weichkaramelle nach einem der Ansprüche 1 bis 14, wobei der Wassergehalt der Weichkaramellengrundmasse 5 bis 14 % Wasser beträgt.

16. Gelatinefreie Weichkaramelle nach Anspruch 15, wobei der Wassergehalt der Weichkaramellengrundmasse 6 bis 12 % Wasser beträgt.

17. Gelatinefreie Weichkaramelle nach einem der Ansprüche 1 bis 16, wobei die Weichkaramellengrundmasse zusätzlich mindestens einen medizinischen Wirkstoff, beispielsweise Dextromethorphan, Hexylresorcin/Menthol, Phenylpropanolamin, Dyclonin, Mentholeukalyptus, Benzocain oder Cetylpyridinium enthält.

18. Gelatinefreie Weichkaramelle nach einem der Ansprüche 1 bis 17, wobei die Weichkaramelle gefüllt oder ungefüllt ist.

19. Gelatinefreie Weichkaramelle nach einem der Ansprüche 1 bis 18, wobei die Weichkaramelle dragiert oder undragiert ist.

20. Verfahren zur Herstellung einer gelatinefreien, Isomaltulose enthaltenden Weichkaramelle, umfassend
a) Herstellung einer nicht-kristallinen Süßungsmittel-Phase durch Lösen mindestens eines löslichen Süßungsmittels in Wasser,
b) Zugabe zu der nicht-kristallinen Süßungsmittel-Phase von
- mindestens einem Polysaccharid-Hydrokolloid, ausgewählt aus der Gruppe bestehend aus Gummi arabicum, Gellan-Gummi, Guar-Gummi, Cellulose-Gummi, Johannisbrotsamen-Gummi, Tamarindensamen-Gummi, Tara-Gummi, Tragant-Gummi, Xanthan-Gummi, Agar, Alginat, Carrageen, Konjak, Pektin, Pullulan, Stärke, modifizierter Stärke und einem Gemisch davon,
- mindestens einem Fettbestandteil;
- mindestens einem Emulgiermittel und
- einem Teil der Gesamtmenge der die kristalline Süßungsmittel-Phase bildenden Isomaltulose;
c) Erhitzen des bei b) erhaltenen Gemisches auf eine Temperatur von mindestens 100°C unter Dampfzufuhr;
d) Zugabe der restlichen Isomaltulose zu dem erhitzten Gemisch unter Rühren;
e) Einführen von Luft in das bei d) erhaltene Gemisch und
f) Kühlen des Gemisches.

21. Verfahren nach Anspruch 20, wobei 70 % bis 90 % der Isomaltulose-Gesamtmenge der nicht-kristallinen Süßungsmittelphase zugegeben und gemeinsam damit erhitzt werden.

22. Verfahren nach Anspruch 21, wobei 74 % bis 85 % der Isomaltulose-Gesamtmenge der nicht-kristallinen Süßungsmittelphase zugegeben und gemeinsam damit erhitzt werden.

23. Verfahren nach einem der Ansprüche 20 bis 22, wobei das die nicht-kristalline Süßungsmittel-Phase enthaltende Gemisch auf 110°C erhitzt wird.

24. Verfahren nach einem der Ansprüche 20 bis 23, wobei nach Erhitzen des die nicht-kristalline Süßungsmittel-Phase enthaltenden Gemisches die Dampfzufuhr beendet und das Gemisch einem Vakuum unterworfen wird.

25. Verfahren nach Anspruch 24, wobei nach Beendigung der Dampfzufuhr die Temperatur des Gemisches auf 125°C bis 130°C steigt.

26. Verfahren nach einem der Ansprüche 20 bis 24, wobei nach Zugabe der restlichen Isomaltulose die Luft durch Aufschlagen des erhitzten Gemisches in dieses eingeführt wird.

27. Verfahren nach einem der Ansprüche 20 bis 25, wobei nach Zugabe der restlichen Isomaltulose das erhitzte Gemisch abgekühlt und Luft durch Ziehen des abgekühlten Gemisches in dieses eingeführt wird.

28. Verfahren nach einem der Ansprüche 20 bis 27, wobei das Luftenthaltende Gemisch nach Abkühlen zu einem Strang gezogen und der Strang in Stücke geschnitten wird.

## Claims

1. Gelatin-free soft caramel comprising a soft caramel base mass that contains
- at least one polysaccharide hydrocolloid selected from the group consisting of gum arabic, gellan gum, guar gum, cellulose gum, carob seed gum, tamarind seed gum, tara gum, gum tragacanth, xanthan gum, agar, alginate, carrageenan, konjac, pectin, pullulan, a starch, a modified starch or a mixture thereof, as texturing agent,
- a crystalline sweetener phase formed by isomaltulose,
- and a noncrystalline sweetener phase.

2. Gelatin-free soft caramel according to claim 1, wherein the polysaccharide hydrocolloid is a mixture of gum arabic and gellan gum.

3. Gelatin-free soft caramel according to claim 2, wherein gum arabic and gellan gum are present in the mixture in a ratio from 5:1 to 15:1.

4. Sugar-free gelatin-free soft caramel according to one of claims 1 to 3, wherein the noncrystalline sweetener phase of the soft caramel base mass is formed of maltitol syrup, polydextrose and/or hydrogenated starch hydrolysate.

5. Sugar-containing gelatin-free soft caramel according to one of claims 1 to 3, wherein the noncrystalline sweetener phase of the soft caramel base mass is formed of glucose syrup and/or starch hydrolysate.

6. Gelatin-free soft caramel according to one of claims 1 to 5, wherein the soft caramel base mass additionally contains one or more intensive sweeteners.

7. Gelatin-free soft caramel according to claim 6, wherein the intensive sweetener is cyclamate, saccharine, aspartame, glycyrrhizin, neohesperidine dihydrochalcone, thaumatin, monellin, acesulfame, alitame or sucralose.

8. Gelatin-free soft caramel according to one of claims 1 to 7, wherein the soft caramel base mass contains 2 to 15% fat.

9. Gelatin-free soft caramel according to one of claims 1 to 8, wherein the soft caramel base mass contains at least one emulsifier.

10. Gelatin-free soft caramel according to one of claims 1 to 9, wherein the soft caramel base mass contains 0 to 5% of at least one protein component, in particular milk protein.

11. Gelatin-free soft caramel according to one of claims 1 to 10, wherein the soft caramel base mass contains one or more natural or synthetic food dyes.

12. Gelatin-free soft caramel according to claim 11, wherein the food dye is chlorophyll, a chlorophyllin, carmine red, alura red, β-carotene, a riboflavin, an anthocyan, betanine, erythrosine, indigo carmine, tartrazine or titanium dioxide.

13. Gelatin-free soft caramel according to one of claims 1 to 12, wherein the soft caramel base mass contains flavorings and flavoring agents.

14. Gelatin-free soft caramel according to claim 13, wherein the flavorings and flavoring agents are essential oils, synthetic flavorings, fruit essences, eucalyptus, peppermint oil, menthol and acids.

15. Gelatin-free soft caramel according to one of claims 1 to 14, wherein the water content of the soft caramel base mass is 5 to 14% water.

16. Gelatin-free soft caramel according to claim 15, wherein the water content of the soft caramel base mass is 6 to 12% water.

17. Gelatin-free soft caramel according to one of claims 1 to 16, wherein the soft caramel base mass additionally contains at least one medicinal active agent, for example dextromethorphan, hexylresorcinol/menthol, phenylpropanolamine, dyclonine, menthol eucalyptus, benzocaine or cetylpyridinium.

18. Gelatin-free soft caramel according to one of claims 1 to 17, wherein the soft caramel is filled or unfilled.

19. Gelatin-free soft caramel according to one of claims 1 to 18, wherein the soft caramel is coated or not coated.

20. Method for producing a gelatin-free isomaltulose containing soft caramel comprised of
a) preparing a noncrystalline sweetener phase by dissolving at least one soluble sweetener in water,
b) adding
- of at least one polysaccharide hydrocolloid selected from the group consisting of gum arable, gellan gum, guar gum, cellulose gum, carob seed gum, tamarind seed gum, tara gum, gum tragacanth, xanthan gum, agar, alginate, carrageenan, konjac, pectin, pullulan, a starch, a modified starch or a mixture thereof,
- at least one fat component,
- at least one emulsifier and
- a part of the total amount of the isomaltulose that forms the crystalline sweetener phase
to the noncrystalline sweetener phase,
c) heating the mixture obtained in (b) to a temperature of at least 100°C by feed of steam,
d) adding the remaining isomaltulose to the heated mixture while stirring,
e) incorporating air into the mixture obtained in (d) and
f) cooling the mixture.

21. Method according to claim 20, wherein 70% to 90% of the total amount of isomaltulose is added to the noncrystalline sweetener phase and which are then heated jointly.

22. Method according to claim 21, wherein 74% to 85% of the total amount of isomaltulose is added to the noncrystalline sweetener phase and which are then heated jointly.

23. Method according to one of claims 20 to 22, wherein the mixture containing the noncrystalline sweetener phase is heated to 110°C.

24. Method according to one of claims 20 to 23, wherein after heating the mixture containing the noncrystalline sweetener phase the feed of steam is stopped and the mixture is subjected to a vacuum.

25. Method according to claim 24, wherein after the end of the feed of steam the temperature of the mixture rises to 125°C to 130°C.

26. Method according to one of claims 20 to 24, wherein after adding the remaining isomaltulose the air is introduced into the mixture by whipping the heated mixture.

27. Method according to one of claims 20 to 25, wherein after adding the remaining isomaltulose the heated mixture is cooled and air is introduced into the mixture by pulling the cooled mixture.

28. Method according to one of claims 20 to 27, wherein the air containing mixture after cooling is pulled to a strand and the strand is cut into pieces.

## Revendications

1. Caramel mou sans gélatine, comprenant une masse de base de caramel mou, qui
- contient au moins un hydrocolloïde à base de polysaccharides, sélectionné dans le groupe consistant en : gomme arabique, gomme gellane, gomme de guar, gomme de cellulose, gomme de graines de caroube, gomme de graines de tamarin, gomme de tara, gomme d'astragale, gomme xanthique, agar-agar, alginate, carraghénine, konjac, pectine, pullulane, amidon, amidon modifié et un mélange de ceux-ci, servant d'agent de texture,
- une phase édulcorante cristalline formée par isomaltulose et
- une phase édulcorante non cristalline.

2. Caramel mou sans gélatine selon la revendication 1, l'hydrocolloïde à base de polysaccharides étant un mélange de gomme arabique et de gomme gellane.

3. Caramel mou sans gélatine selon la revendication 2, la gomme arabique et la gomme gellane présentant un rapport de 5:1 à 15:1 dans le mélange.

4. Caramel mou sans gélatine ni sucre selon l'une des revendications 1 à 3, la phase édulcorante non cristalline de la masse de base de caramel mou étant formée à partir de sirop de maltitol, de polydextrose et/ou d'hydrolysat d'amidon hydrogéné.

5. Caramel mou sans gélatine contenant du sucre selon l'une des revendications 1 à 3, la phase édulcorante non cristalline de la masse de base de caramel mou étant formée à partir de sirop de glucose et/ou d'hydrolysat d'amidon.

6. Caramel mou sans gélatine selon l'une des revendications 1 à 5, la masse de base de caramel mou contenant en outre un ou plusieurs agents édulcorants intenses.

7. Caramel mou sans gélatine selon la revendication 6, l'agent édulcorant intense étant du cyclamate, de la saccharine, de l'aspartame, de la glycyrrhizine, de la néohespéridine dihydrochalcone, de la thaumatine, de la monelline, de l'acésulfame, de l'alitame ou du sucralose.

8. Caramel mou sans gélatine selon l'une des revendications 1 à 7, la masse de base de caramel mou contenant de 2 à 15 % de lipides.

9. Caramel mou sans gélatine selon l'une des revendications 1 à 8, la masse de base de caramel mou contenant au moins un agent émulsifiant.

10. Caramel mou sans gélatine selon l'une des revendications 1 à 9, la masse de base de caramel mou contenant de 0 à 5 % au moins d'un constituant protéique, en particulier de la protéine de lait.

11. Caramel mou sans gélatine selon l'une des revendications 1 à 10, la masse de base de caramel mou contenant un ou plusieurs colorants alimentaires naturels ou synthétiques.

12. Caramel mou sans gélatine selon la revendication 11, le colorant alimentaire étant de la chlorophylle, une chlorophylline, du rouge carmin, du rouge allura, du bêta-carotène, une riboflavine, une anthocyanine, de la bêtanine, de l'érythrosine, du carmin d'indigo, de la tartrazine ou du dioxyde de titane.

13. Caramel mou sans gélatine selon l'une des revendications 1 à 12, la masse de base de caramel mou contenant des arômes et agents aromatisants.

14. Caramel mou sans gélatine selon la revendication 13, les arômes et agents aromatisants étant des huiles essentielles, des arômes artificiels, des essences de fruits, de l'eucalyptus, de l'huile de menthe poivrée, du menthol et des acides.

15. Caramel mou sans gélatine selon l'une des revendications 1 à 14, la teneur en eau de la masse de base de caramel mou étant de 5 à 14 % d'eau.

16. Caramel mou sans gélatine selon la revendication 15, la teneur en eau de la masse de base de caramel mou étant de 6 à 12 % d'eau.

17. Caramel mou sans gélatine selon l'une des revendications 1 à 16, la masse de base de caramel mou contenant en outre au moins une substance active médicinale, par exemple dextrométhorphane, hexylrésorcine/menthol, phénylpropanolamine, dyclonine, menthol eucalyptus, benzocaïne ou cétylpyridinium.

18. Caramel mou sans gélatine selon l'une des revendications 1 à 17, le caramel mou comportant ou non une matière de remplissage.

19. Caramel mou sans gélatine selon l'une des revendications 1 à 18, le caramel mou étant ou non dragéifié.

20. Procédé de fabrication d'un caramel mou sans gélatine contenant de l'isomaltulose, comprenant
a) la fabrication d'une phase édulcorante non cristalline par dissolution au moins d'un agent édulcorant soluble dans l'eau,
b) l'addition à la phase édulcorante non cristalline de
- au moins un hydrocolloïde à base de polysaccharides, sélectionné dans le groupe consistant en gomme arabique, gomme gellane, gomme de guar, gomme de cellulose, gomme de graines de caroube, gomme de graines de tamarin, gomme de tara, gomme d'astragale, gomme xanthique, agar-agar, alginate, carraghénine, konjac, pectine, pullulane, amidon, amidon modifié et un mélange de ceux-ci,
- au moins un constituant lipidique ;
- au moins un agent émulsifiant et
- une partie de la quantité totale de l'isomaltulose formant la phase édulcorante cristalline ;
c) le chauffage du mélange obtenu par b) à une température d'au moins 100 °C sous alimentation en vapeur ;
d) l'addition de l'isomaltulose restante au mélange chauffé tout en agitant ;
e) l'introduction d'air dans le mélange obtenu par d) et
f) le refroidissement du mélange.

21. Procédé selon la revendication 20, dans lequel 70 % à 90 % de la quantité totale d'isomaltulose de la phase édulcorante non cristalline sont ajoutés puis chauffés ensemble.

22. Procédé selon la revendication 21, dans lequel 74 % à 85 % de la quantité totale d'isomaltulose de la phase édulcorante non cristalline sont ajoutés puis chauffés ensemble.

23. Procédé selon l'une des revendications 20 à 22, dans lequel le mélange contenant la phase édulcorante non cristalline est chauffé à 110 °C.

24. Procédé selon l'une des revendications 20 à 23, dans lequel, après chauffage du mélange contenant la phase édulcorante non cristalline, il est mis fin à l'alimentation en vapeur et le mélange est mis sous vide.

25. Procédé selon la revendication 24, dans lequel la température du mélange augmente jusqu'à 125 °C à 130 °C après l'arrêt de l'alimentation en vapeur.

26. Procédé selon l'une des revendications 20 à 24, dans lequel, après addition de l'isomaltulose restante, l'air est introduit dans le mélange chauffé par fouettage de ce dernier.

27. Procédé selon l'une des revendications 20 à 25, dans lequel, après addition de l'isomaltulose restante, le mélange chauffé est refroidi et l'air est introduit dans le mélange refroidi par étirage de ce dernier.

28. Procédé selon l'une des revendications 20 à 27, dans lequel le mélange contenant de l'air est extrait en un fil après refroidissement et le fil est coupé en morceaux.
